# EUROPEAN PATENT SPECIFICATION

(11) **EP 1 802 324 B1**
(45) Date of publication and mention of the grant of the patent: **02.06.2010**
(21) Application number: 05787236.8
(22) Date of filing: 22.09.2005
(51) Int. Cl.: A61K 38/17, A61P 3/10

(54) **INHIBITORS OF TASTE RECEPTORS FOR USE IN THE TREATMENT OF OBESITY AND DIABETES**
INHIBITOREN DER GESCHMACKSREZEPTOREN ZUR VERWENDUNG IN DER BEHANDLUNG VON FETTLEIBIGKEIT UND ZUCKERKRANKHEIT
INHIBITEURS DES RÉCEPTEURS DU GOÛT POUR L'UTILISATION DANS LE TRAITEMENT DE L'OBÉSITÉ ET DU DIABÈTE

(30) Priority: 22.09.2004 EP 04077610
(43) Date of publication of application: 04.07.2007
(73) Proprietor: VIB, vzw, 9052 Zwijnaarde (BE)
(72) Inventor: SHIRAZI-BEECHEY, Soraya, Wirral CH48 1NF (GB); DYER, Jane, Liverpool L12 0BG (GB)
(86) International application number: PCT/EP2005/054760
(87) International publication number: WO 2006/032693

(56) References cited:
- HÖFER ET AL: "Taste receptor-like cells in the rat gut identified by expression of alpha-gustducin" PROCEEDINGS OF THE NATIONAL ACADEMY OF SCIENCES; USA, vol. 93, 1996, pages 6631-6634, XP002312297
- HÖFER ET AL: "Chemosensory perception in the gut" NEWS IN PHYSIOLOGICAL SCIENCES, vol. 14, 1999, pages 18-23, XP002299243
- WU ET AL: "Expression of bitter taste receptors of the T2R family in the gastrointestinal tract and enteroendocrine STC-1 cells" PROCEEDINGS OF THE NATIONAL ACADEMY OF SCIENCES, USA, vol. 99, 2002, pages 2392-2397, XP002982185
- DYER ET AL: "Glucose sensing in the intestinal epithelium" EUROPEAN JOURNAL OF BIOCHEMISTRY, vol. 270, 2003, pages 3377-3388, XP002360203 cited in the application
- CHAUDHARI ET AL: "Molecular basis of the sweet tooth" THE LANCET, vol. 358, 2001, pages 2101-2102, XP004805187
- GROTZ ET AL: "Lack of effect of sucralose on glucose homeostasis in subjects with type 2 diabetes" JOURNAL OF THE AMERICAN DIETETIC ASSOCIATION, vol. 103, 2003, pages 1607-1612, XP004591974
- DYER ET AL: "Expression of sweet taste receptors of the T1R family in the intestinal tract and enteroendocrine cells" BIOCHEMICAL SOCIETY TRANSACTIONS, PART I, vol. 33, February 2005 (2005-02), pages 302-305, XP002360204
- TANIGUCHI ET AL: "Expression of the sweet receptor protein, T1R3, in the human liver and pancreas" JOURNAL OF VETERINARY MEDICAL SCIENCE, vol. 66, 2004, pages 1311-1314, XP001091176

## Description

The present invention relates to mammalian intestinal epithelial glucose sensors, more specifically to human intestinal epithelial glucose sensors.

In particular, the invention refers to the use of certain inhibitors of the T1 R2 and/or T1 R3 receptor and/or α-gustducin for the manufacture of a medicament to treat obesity and diabetes.

The invention also refers to the use of intestinal epithelial cells expressing the T1 R2 and/or T1 R3 receptor and/or α-gustducin to screen for compounds for preparing such medicaments.

Sensing nutrients is a fundamental challenge for all living cells. Different types of nutrient sensing receptors have been identified in eukaryotic cells. Sensing nutrients is particularly important for the absorptive cells of the intestinal epithelium. These cells are exposed to a luminal environment that varies considerably with diet, and not surprisingly therefore, they adapt to these changes by regulating their uptake of nutrients from the intestinal lumen (Karasov and Diamond, 1987; Ferraris and Diamond, 1989). Although it is well established that this adaptation is achieved through the modulation of expression/activity of specialised nutrient transporters resident in the enterocyte plasma membrane, a major challenge that remains is to gain an insight into the identity of the receptors that sense the changes in the luminal contents; i.e. the nutrient sensors.

The best example of adaptive response of intestinal nutrient transport to the changes in the luminal nutrients is that of the intestinal Na⁺/glucose cotransporter, SGLT1. SGLT1 transports dietary monosaccharides, D-glucose and D-galactose from the lumen of the intestine across the luminal membrane (brush border membrane) into enterocytes. Using both *in vivo and in vitro* models it has been shown that the activity and the expression of SGLT1 is directly regulated by the luminal (medium) monosaccharides, and that the metabolism of glucose is not required for the glucose induction of SGLT1 (Ferraris and Diamond, 1989; Solberg and Diamond, 1987; Lescale-Matys *et al*., 1993; Shirazi-Beechey, 1996; Dyer *et al*., 1997). Furthermore a membrane impermeable glucose analogue, when introduced into the lumen of the intestine, also stimulates SGLT1 expression and abundance, implying that a glucose sensor expressed on the luminal membrane of the intestinal cells is involved in sensing the luminal sugar (Dyer, J and Vayro S (joint first) *et al*., 2003).

The intestinal epithelium is a dynamic structure, undergoing constant and rapid renewal. The stem cells positioned near the base of the crypt undergo several rounds of cell division and give rise to four cell types, absorptive enterocytes, mucous producing goblet cells, hormone producing enteroendocrine cells, and paneth cells. Enterocytes, constituting 90% of cells, along with goblet and some endocrine cells migrate without subsequent division to the villus tip, where they are extruded into the lumen of the intestine. This process takes 3-4 days.

It is well established that SGLT1 is expressed on the luminal membrane throughout the entire villus enterocytes. It is not known, however, which cell type(s) may express the glucose sensor. Generally it is accepted that enteroendocrine cells possess nutrient sensing properties, and secrete gut hormones in response to luminal nutrients. As such enteroendocrine cells may be the cell type that expresses the glucose receptor.

The only knowledge of sugar sensing in the mammalian gastrointestinal tract is from taste transduction mechanisms. Taste cells in the taste buds of the tongue epithelium have mechanisms that can distinguish chemical compounds, such as sugars, having potential nutritional value. It has been shown that transduction of sweet-tasting compounds involves activation of G-protein coupled receptor (GPCR) on the apical surface of taste receptor cells. Recent studies indicate that the members of the taste T1R receptor family (T1R2/T1R3) and gustducin, a taste-specific transducin-like G-protein a subunit, are involved in transduction of sugars in the tongue. Although expression of α-gustducin has been shown in rat gut (Höfer et al. 1996) expression of the sweet receptor protein T1R3 has been reported in several tissues (Kitagawa et al. 2001; Max et al 2001) but not in the intestine.

Surprisingly we found that taste receptors, T1R1-3 are expressed in the small intestine. Furthermore we demonstrate that the receptors along with Gα_{gust} are expressed luminally, and mainly in the proximal region of the small intestine. As these GPCRs are involved in sensing dietary glucose their manipulation will result in modulation in the capacity of the gut to absorb dietary sugars. This has both nutritional and clinical significance, in the treatment of, as a non-limiting example, obesity and diabetes.

A first aspect of the invention is the use of an inhibitor of T1 R2 and/or T1 R3, preferably an inhibitor of T1R3, to modulate sugar uptake in the intestine. Preferably, said modulation of the sugar uptake is realized by a modulation of the activity of the SGLT1 transporter. The inhibitor of T1 R2 and/or T1R3 inhibits ligand binding, secretion and/or localization into the plasma membrane, clustering of the receptor, posttranslational modification, dimerization and/or the signalling which is normally induced upon binding of the ligand to the receptor. Preferably, said inhibitor is interfering with the ligand binding. In a first embodiment, the inhibitor is an antibody binding to the ligand binding domain of the receptor. An antibody, as used here can be any antibody known to the person skilled in that art, including, but not limited to single chain antibodies and camelid antibodies and any derived nanobodies. In a second embodiment, the inhibitor is a soluble peptide or a peptido-mimetic comprising the ligand binding domain of the receptor. Indeed, such a compound will bind the ligand and act as a competitive inhibitor of the receptor. In the third embodiment, the inhibitor is an inhibitor of the signalling pathway, namely a G352P-α-gustducin mutant, which acts as a dominant negative mutant (Ruiz-Avila *et al*., 2001).

Another aspect of the invention is the use of an isolated intestinal epithelial cell expressing T1R2 and/or T1R3 and/or α-gustducin, preferably an isolated intestinal epithelial cell expressing T1R3 and/or α-gustducin to screen for compounds for the manufacture of a medicament to treat obesity and/or diabetes. Intestinal epithelial cell lines are known to the person skilled in the art and include, but are not limited to the primary human small intestinal epithelial cell line (FHs74 Int), primary rat small intestinal cell line (IEC6), and Caco-2 cells (human colon carcinoma cell line, widely used as a model of small intestinal cell). Preferably, said isolated intestinal epithelial cell is STC-1 cell or GLUtag cell. As a non-limiting example, said screening can be performed by placing a reporter gene under control of a T1 R2 and/or T1R3 responsive promoter. A *Reporter gene* as used here means any gene that leads to a detectable signal and can be, as a non-limiting example, an antibiotic resistance gene, a toxin gene resulting in cell death, a gene encoding a fluorescent protein such as GFP, or a gene encoding an enzyme activity such, as β-galactosidase. The coding sequence is placed under control of a T1 R2 and/or T1R3 responsive promoter, i.e. a promoter that is induced by binding of a ligand to the receptor and consequent induction of the signalling pathway. The induction of the reporter may be direct or indirect. A direct induction means that the reporter gene is induced by the signalling pathway, which is activated upon binding of the ligand to the receptor. An indirect induction means that, upon binding of the ligand to the receptor, an intermediate compound is synthesized by the cell, which is secreted and activates a second receptor situated either on the same cell or on another cell type, whereby the activation of the second receptor will induce the reporter gene.

Preferably, said T1R2 and/or T1R3 responsive promoter is the SGLT1 promoter. A preferred embodiment is a screening system, comprising (a) exposing cells expressing T1R family members and/or Gα_{gust} (cell A) to a defined level of glucose, which is activating the receptor; (b) contacting said cells with a possible inhibitor or activator (c) removing samples of media and treat the culture of enterocytes comprising a reporter gene operably linked to the SGLT1 promoter (cell B). The read out is the expression of the reporter gene in the enterocytes (cell B) in response to the presence of the inhibitor or activator, using the normal induction (without addition of an inhibitor or activator) as control.

### BRIEF DESCRIPTION OF THE FIGURES

**Figure 1****: Expression of Na⁺/glucose cotransporter (SGLT1), T1R family members and α-gustducin along the mouse intestinal crypt-villus axis.** Real-time PCR was performed on cDNA (50 ng per reaction) synthesised from total RNA isolated from upper villus, lower villus and crypt cell fractions. T1R2/T1R3 (sweet taste receptors), T1R1 (a component of the umami (savoury) taste receptor).
**Figure 2****: Expression of SGLT1, T1R2, T1R3 and Gα_{gust} in the small intestine.** Western blot analysis was performed on brush-border membrane vesicles (40 µg protein per lane) isolated from the mouse duodenum (D), jejunum (J) and ileum (I).
**Figure 3****: Expression of proteins along the crypt-villus axis of the mouse small intestine.** (A) Western blot analysis was performed on post-nuclear membrane proteins (30 µg per lane) isolated from upper villus (UV), lower villus (LV) and crypt (C) cells. (B) Densitometric analysis of western blot data normalised to β-actin.
**Figure 4****: *In situ* hybridisation histochemistry of T1Rs and α-gustducin in mouse proximal small intestine.**
   Sections of mouse proximal small intestine were treated as described in the methods and hybridised with digoxigenin-labelled anti-sense riboprobes to unique sequences of the T1R1, T1R2 and T1R3 taste receptors and α-gustducin protein coding regions. Signals were developed using NBT/BCIP and sections were counter-stained with methyl green. Scale bars represent 20 µm.

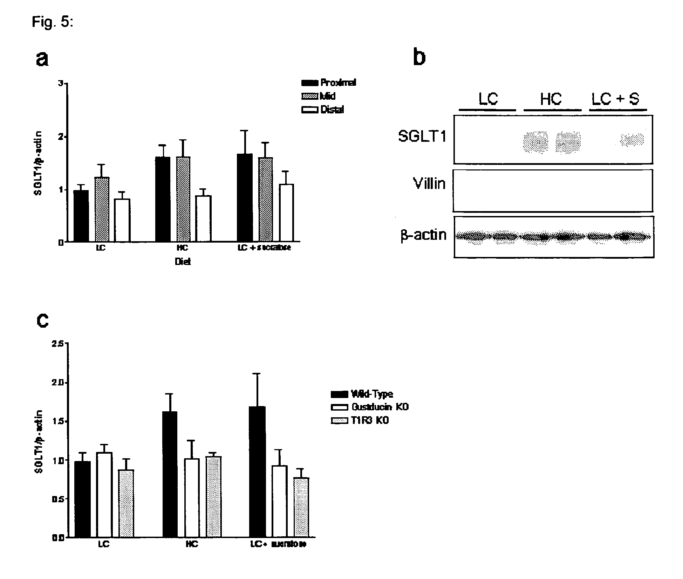

**Figure 5****: The effect of dietary carbohydrate level on SGLT1 expression in the small intestine of wild-type, α-gustducin and T1R3 KO mice. a,** Real-time PCR data of SGLT1 mRNA levels, normalised to β-actin, in wild-type mouse proximal, mid, and distal intestine maintained on low carbohydrate (LC), high carbohydrate (HC) and LC plus sucralose diets for 2 weeks. Data are mean ± S.E.M. (n = 4). **b**, Representative western blot analysis of luminal membrane vesicles isolated from the proximal intestine of wild-type mice. **c**, Real-time PCR data of SGLT1 expression in the proximal intestine of wild-type and KO mice in response to diet. Data are mean ± S.E.M. (n = 4).

### EXAMPLES

### Materials and Methods to the examples

### Animals and tissue collection.

Male CD-1 and C57BL/6 mice, six weeks old, from Charles River Laboratories were used. The α-gustducin knock out mouse was described by Wong *et al*. (1996); the T1R3 knock out mouse was described by Damak *et al*. 2003).

High and low carbohydrate diets were resp. TestDiet^{®} 5810 and TestDiet^{®} 5787-9. For the sucralose test, the low carbohydrate diet was supplemented with sucralose (1,6-dichloro-1,6-dideoxy-beta-D-fructofuranosyl-4-chloro-4-deoxy-alpha-galactopyranoside) at 2mM.

Animals were killed by concussion followed by cervical dislocation. The entire small intestine was removed and flushed with ice-cold 0.9% NaCl, opened longitudinally, rinsed in saline and mucous removed by blotting. The small intestine was then divided into proximal, mid and distal sections and the mucosa removed by scraping. Mucosal scrapings were frozen immediately in liquid nitrogen and stored at -80°C until use.

Sections (1 cm) for immunohistochemistry and *in situ* hybridisation histochemistry were placed in PBS plus 4% paraformaldehyde.

For investigation of expression along the crypt-villus axis cell populations were removed by the technique of Meddings *et al*. (1990) adapted for use at 4°C.

As positive controls mouse tongues were removed and the epithelium dissected away from the muscle and frozen immediately in liquid nitrogen, or placed in PBS plus 4% paraformaldehyde.

### Reverse Transcription PCR.

RNA was isolated from intestinal mucosal scrapings using the Qiagen RNeasy Mini Kit with on-column DNase 1 digestion. Poly (A⁺) RNA was isolated from total RNA using the Qiagen mRNA isolation kit. RT-PCR was performed on 25 ng of mRNA in a single tube reaction with primers designed to homologous regions of the mouse, rat and human sweet taste GPCRs T1R1, T1 R2, T1R3 and the G-protein α-gustducin (Gα_{gust}). PCR products were cloned into pGEM-T and sequenced. CLUSTALW alignment of the DNA sequences was performed using Vector NTi Suite (Informax).

### Real-time PCR.

Using the Primer Express software programme (Applied Biosystems) PCR primers and probes (FAM/TAMRA labelled) for the amplification of T1R1, T1R2, T1R3, Gα_{gust}, and the Na⁺/glucose co-transporter (SGLT1), along with β-actin (JOE/TAMRA labelled) were designed. Primers and probes were purchased from Eurogentec, along with 18S ribosomal RNA controls.

cDNA was synthesised from either total RNA or mRNA using Supercript III reverse transcriptase (Invitrogen) and either oligo(dT)₁₂₋₁₈ or random primers, cleaned up using the Machery-Nagel Nucleospin extract kit and 50ng of cDNA used per reaction.

For Real-Time PCR reactions the enzyme was activated by heating at 95°C for 2 min. A two-step PCR procedure was used, 15 s at 95°C and 60 s at 60°C for 45 cycles in a PCR mix containing 5 µl of cDNA template, 1X Jumpstart qPCR master mix (Sigma-Aldrich), 900 nM of each primer and 250 nM probe in a total volume of 25 µl. Where multiplex reactions were performed the β-actin primers were primer limiting and used at 600 nM. All reactions were performed in a RotorGene 3000 (Corbett Research).

### Western blotting.

Brush-border membrane vesicles were isolated from intestinal mucosal scrapings and isolated cells by the cation precipitation, differential centrifugation technique described previously (Shirazi-Beechey et al. 1990). Membrane proteins were denatured in SDS-PAGE sample buffer (20 mM Tris/HCl, pH 6.8, 6% SDS, 4% 2-mercaptoethanol and 10% glycerol) by heating at 95°C for 4 min and were separated on 8% polyacrylamide gels and electrotransferred to PVDF membranes. Membranes were blocked by incubation in TTBS plus 5% non-fat milk for 60 min. Membranes were incubated for 60 min with antisera to SGLT1, T1 R2 (Santa-Cruz), T1R3 (AbCam), Gα_{gust} (Santa-Cruz), villin (The Binding Site), and β-actin (Sigma-Aldrich) in TTBS containing 0.5% non-fat milk. Immunoreactive bands were visualised by using horseradish peroxidase-conjugated secondary antibodies and enhanced chemiluminescence (Amersham Biosciences). Scanning densitometry was performed using Phoretix 1D (NonLinear Dynamics).

### Immunohistochemistry.

Tissue sections (fixed for 6 hours in 4% (w/v) paraformaldehyde in PBS) were paraffin wax-embedded and sectioned at a thickness of 5-7µm onto Poly-L-lysine -coated slides.

Slides were then de-waxed as follows: 3 x 5 minutes in xylene; 2 x 5 minutes in absolute ethanol, 2 x 5 minutes in 70% (w/v) ethanol, 2 x 5 minutes in dd H₂O. Washes, 2 x 5 minute, in PBS were performed before antigen retrieval by autoclaving in 10mM Tris buffer (pH 10) for 11 minutes. A further 2 x 5 minute washes were performed and then endogenous H₂O₂ was blocked by incubation in 3% H₂O₂ / PBS for 15 minutes. Another 2 x 5 minute washes in PBS were carried out and then a 1 hour incubation at room temperature in a humidity chamber in 5% BSA / PBS to block non-specific protein-binding sites in the tissue sections.

The slides were then incubated in primary antibody diluted in 1% BSA / PBS (1:50 for both α-gustducin and T1R2) at room temperature overnight in a humidity chamber. 3 x 5 minute washes in PBS were performed prior to incubation in HRP-conjugated swine anti-rabbit secondary antibody (DAKO) for α-gustducin or HRP-conjugated rabbit anti-goat for T1 R2, diluted 1:200 in 1% BSA / PBS for 1 hour at room temperature in a humidity chamber. A further 3 x 5 minute washes were performed and then the slides were developed in 0.05% DAB / 0.03% H₂O₂ / 0.05M Tris-HCl pH 7.6 for 2-10 minutes at room temperature in a humidity chamber in the dark.

The slides were then counterstained in 1% chloroform-extracted methyl green for 5 minutes. The dye was rinsed off in running tap water, slides allowed to slowly air dry and were then mounted / cover-slipped using DPX (Raymond Lamb).

### In situ hybridisation histochemistry

Tissue sections of varying fixation times (12-48 hours) were paraffin wax-embedded and microtomed at a thickness of 5-7µm onto APES or Poly-L-lysine -coated slides. Slides were then de-waxed in xylene and then rehydrated through graded ethanol to dd H₂O.

The tissue was then permeabilised as follows: 20 min wash in 200mM HCl; 2 x 3 min washes in 2X SSC; 3 min equilibration in Proteinase K Buffer (0.05M Tris / HCl pH 7.4); 1 hour incubation at 37°C in Proteinase K Buffer containing 0-10µg/ml (determined empirically) Proteinase K (Sigma); 2 x 3 minute washes in 0.2% Glycine / PBS; rinse in PBS.

Anticipated background was reduced as follows: 3 min equilibration in 0.1M Triethanolamine pH 8.0; 10 min wash in 0.1M Triethanolamine pH 8.0 containing 0.25% (v/v) acetic anhydride (added fresh); rinse in PBS; post-fixation in 4% paraformaldehyde / PBS; 1 min block of endogenous alkaline phosphatase in 20% acetic acid; rinse in PBS.

The slides were then pre-hybridised in a hybridisation buffer (50% de-ionised formamide, 300mM NaCl, 20mM Tris/HCl pH 8.0, 5mM EDTA, 1X Denhardt's, 1X RNA Protect (Sigma), 100 mg/ml dextran sulphate) for 1 hour at 60°C. The slides were then hybridised overnight at 50°C in hybridisation buffer containing 100µg/ml tRNA and 50-500 ng/ml probe (determined empirically).

After hybridisation, the following stringency washes were performed: 1 hour wash in 2X SSC; 4 hour wash at 50°C in Riboprobe Wash Buffer (300mM NaCl, 200mM Tris/HCl pH 8.0, 10mM EDTA, 50% formamide, 1X Denhardt's); overnight wash at 50°C in Riboprobe Wash Buffer; 30 min wash in 2X SSC; 30 min wash in 0.1X SSC.

The slides were then subjected to the following detection procedure: 5 min equilibration in DIG-AP Buffer (100mM Tris / HCl pH 7.5, 150mM NaCl); 30 min block in DIG-AP Buffer containing 0.5% (w/v) DIG Blocking Reagent (Roche); 2 hour incubation in DIG-AP Buffer containing 0.5% (w/v) DIG Blocking Reagent (Roche) and anti-DIG AP-conjugated antibody diluted 1:1000; 5 min wash in DIG-AP Buffer; 5 min equilibration in NBT/BCIP Buffer (100mM Tris / HCl pH 9.5, 100mM NaCl); 1-24 hour incubation (determined empirically) in the dark in NBT/BCIP Buffer containing NBT/BCIP Mixture (Roche) diluted 1:50; 5 min wash in 10mM Tris / HCl pH 8.0, 1 mM EDTA.

Slides were then rinsed in tap water, counter-stained in chloroform-extracted 1% methyl green for 5 minutes, washed in tap water, slowly air dried and then cover-slipped and mounted in Glass Bond (Loctite). Slides were viewed using a Nikon Eclipse 400 microscope and images captured with a Nikon DXM1200 digital camera.

### Example 1: Expression of sweet taste receptors in the murine small intestinal mucosa as determined by RT-PCR

To examine T1R expression in the small intestine RT-PCR was performed on mRNA isolated from mucosal scrapings of the proximal small intestine of CD-1 mice using specific primers based on the mouse, rat and human sequences. PCR products of the predicted size, 1127 bp for T1R1, 756 bp for T1R2, 855 bp for T1R3 and 900 bp for Gα_{gust} were cloned and sequenced. Sequence analysis confirmed that all were 100% homologous to the reported mouse sequences cloned from taste-buds on the tongue. This indicates that taste receptors are expressed in the proximal part of the small intestine.

### Example 2: Expression of sweet taste receptors in the murine small intestinal mucosa as determined by Real-time PCR

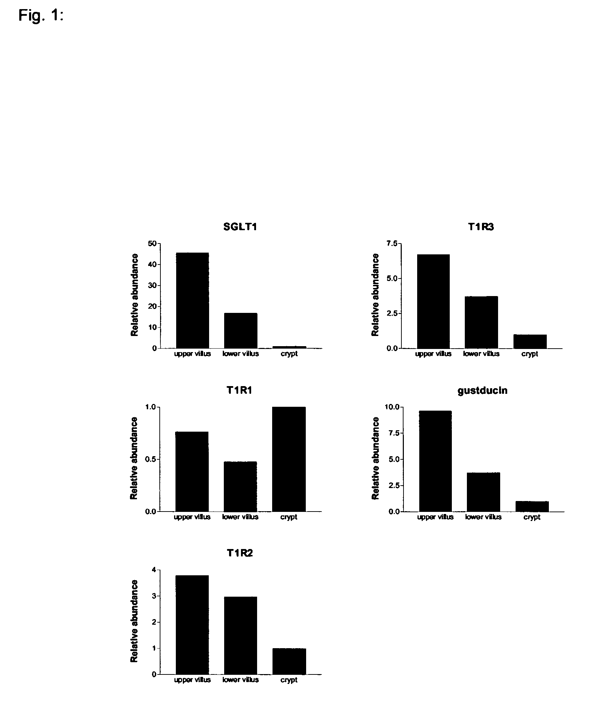

To determine the expression of the T1R family members and Gα_{gust} throughout the small intestine, and along the crypt-villus axis, the technique of real-time PCR was used with primers and probes designed specifically to detect mouse T1R1, T1R2, T1R3 and Gα_{gust}. Results indicated that all members of the T1R family and gustducin are expressed along the length of the small intestine. Expression levels were low (equivalent to those seen in the tongue) and suggested that the receptors are expressed in only a sub - population of cells rather than in all intestinal cells along the crypt-villus axis (this conclusion is supported by immunohistochemical data). Expression patterns along the crypt-villus axis indicated that expression of T1 R2/T1 R3 (receptors known to taste sweets) was higher in the villus cell fractions than in the crypts. T1R1 (a component of the umami taste receptor) appear to have a different pattern of expression (see Figure 1).

### Example 3: Expression of sweet taste receptors in the murine small intestinal mucosa as determined by Western blotting

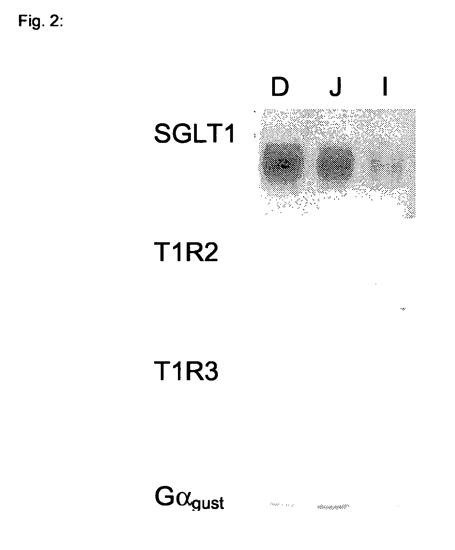

Expression of the proteins was investigated using the commercially available antibodies to T1R2, T1R3 and Gα_{gust}. Western blotting indicated that the antibodies to T1R2 and T1R3 each identified a single protein in the purified intestinal brush-border membrane vesicles which was present in the proximal mid and distal small intestinal fractions, with slightly higher levels in the mid small intestine. The Gα_{gust} antibody identified two bands of approximately 55 kDa and 110 kDa in the brush-border membrane vesicles from proximal, mid and distal intestine, again with slightly higher levels in the mid small intestine. All data was normalised to the expression of β-actin. This indicates that Gα_{gust}, T1 R2 and T1 R3 are expressed on the luminal membrane of gut cells, with higher expression in the jejunum (see Figure 2).

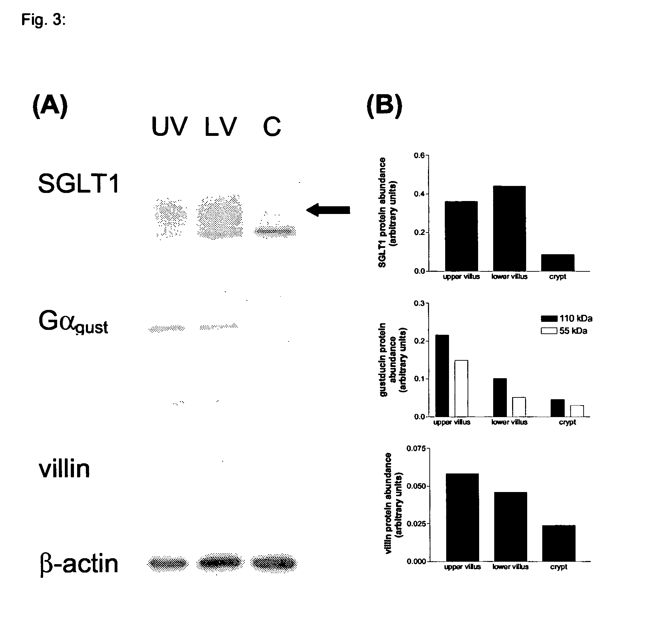

Cells isolated from along the crypt-villus axis were western blotted for the expression of SGLT1 and villin, well characterised markers of enterocyte differentiation. The crypt-villus expression of these markers was as previously reported and indicated that the cell fractions were derived from the expected upper villus, mid-to-lower villus and crypt regions. Gα_{gust} expression along the crypt-villus axis indicated that protein expression increased towards the upper part of the villus being lowest in the crypt and correlated with the Gα_{gust} mRNA expression determined by real-time PCR (see Figure 3).

### Example 4: T1R and α-gustducin are expressed in human small intestine; T1Rs and α-gustducin proteins are associated with the luminal membrane.

Using real time quantitative PCR and western blot analysis we have demonstrated the presence of T1R1, T1 R2, T1 R3 and α-gustducin throughout the human small intestine. T1Rs and α-gustducin proteins are associated with the luminal membrane. Very low levels of T1Rs and α-gustducin are also detected in human colon

### Example 5: Expression of T1Rs and α-gustducin along crypt-villus axis of mouse intestine.

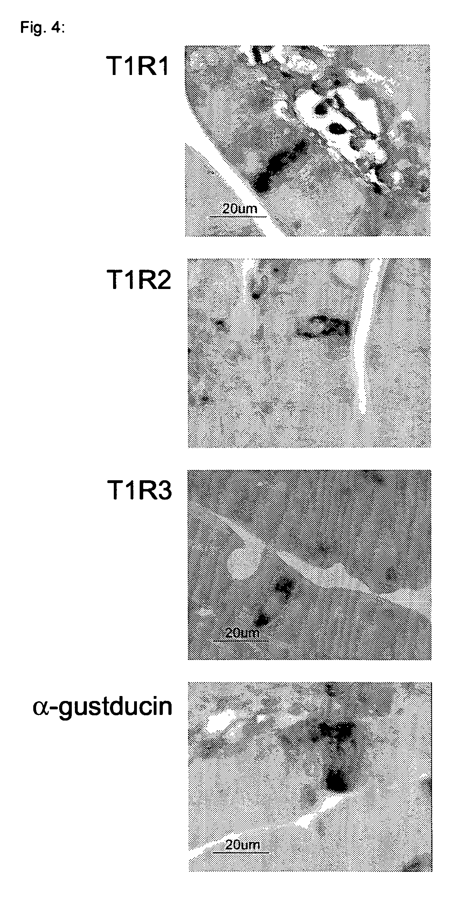

Employing techniques of *in situ* hybridisation and immunohistochemistry, we have shown that T1Rs and α-gustducin are co-expressed at protein and mRNA levels in a sub-population of cells along the crypt-villus axis of mouse small intestine rather than being expressed in the entire enterocyte population. The T1R and α-gustducin proteins are associated with the luminal membrane of the same cells. Typical results of in situ hybridization are shown in figure 4. The co-expression of T1Rs and α-gustducin in same cell population strengthen their association. The fact that they are expressed in a sub - population of cells, and not the entire enterocyte population, highlights the presence of specific sensor cells. The demonstration that T1Rs and α-gustducin are associated with the luminal membrane of gut cells reinforces their role in luminal sensing.

### Example 6: T1R3 and α-gustducin Knock Out mice are affected in the regulation of SGLT1 expression by carbohydrate.

To investigate any direct links between T1Rs, α-gustducin, and SGLT1 expression, we performed dietary trials on T1R3^{-/-} and α-gustducin^{-/-} knock-out mice.

Firstly, groups of wild-type and T1 R3 and α-gustducin "knock-out" (KO) mice (Damak et al. 2003; Wong et al. 1996) were placed on standard diets with the same carbohydrate composition for two weeks. After this time the mice were killed and the small intestine removed, divided into proximal, mid and distal regions, and SGLT1 expression at the levels of mRNA and protein was measured. The rates of glucose transport were also determined in brush-border membrane vesicles isolated from the tissues.

There were no differences in the levels of SGLT1 mRNA, SGLT1 protein and glucose transport in the intestine of wild-type and KO mice. Therefore all animals had the capacity to absorb dietary sugars. This was evident since neither groups showed any signs of intestinal malabsorption.

Second, groups of wild-type and T1R3 and α-gustducin KO mice were placed on each of three iso-caloric diets a) low carbohydrate, b) high carbohydrate, and c) low carbohydrate + artificial sweetener (sucralose), for two weeks. After this time the mice were killed and the small intestines were removed, divided into proximal, mid and distal regions, and SGLT1 expression, at protein and mRNA levels, were measured in each. The results are shown in Figure 5. Figure 5A shows the changes in SGLT1 mRNA levels, measured by qPCR in wild-type mice. SGLT1 mRNA is increased 30-70% in the proximal and mid intestinal regions in response to both the high carbohydrate diet and the addition of sucralose to the low carbohydrate diet. Increased SGLT1 expression in mice in response to an increase in dietary carbohydrate has been reported previously, and is a well established phenomenon (Ferraris and Diamond 1989). The increase in SGLT1 expression in response to sucralose is a novel finding. Sucralose is marketed as a compound that has no physiological effect on the body other than a sweet taste. It is reported to be non-hydrolysed, non-transported and non-metabolised within the mammalian small intestine (Roberts et al. 2000).

Our data show that SGLT1 protein expression is also increased in response to both high carbohydrate and low carbohydrate + sucralose diets (Figure 5B) in wild-type animals.

In contrast to the wild type situation, there was no increase in SGLT1 mRNA and protein in response to high carbohydrate and low-carbohydrate + sucralose diets in both T1R3 and α-gustducin KO animals (Figure 5C) proving that both T1R3 and α-gustducin are required for this response as key components of the intestinal sugar-sensor. This novel finding supports our proposition that the taste receptor T1 R3 and the G-protein α-gustducin are constituents of the intestinal glucose sensing mechanism which ultimately results in the modulation of SGLT1 expression and the capacity of the small intestine to absorb sugars.

### REFERENCES

- Damak, S., Rong, M., Yasumatsu, K., Kokrashvili, Z., Varadarajan, V., Zou, S., Jiang, P., Ninomiya, Y. and Margolskee, R.F. (2003). Detection of sweet and umami taste in the absence of taste receptor T1 R3. Science, 301, 850-853.
- Dyer, J., Hosie, K.B. and Shirazi-Beechey, S.P. (1997). Nutrient regulation of human intestinal sugar transporter (SGLT1) expression. Gut, 41, 56-59.
- Dyer, J., Vayro, S. (joint first), King, T.P. and Shirazi-Beechey, S.P. (2003). Glucose sensing in the intestinal epithelium. Eur. J. Biochem. 270, 1-12.
- Dyer, J. et al. (2005) Expression of the sweet taste receptors of the TIR family in the intestinal tract and entero-endocrine cells. Biochem. Soc. Trans, 33, 302-305.
- Ferraris, R.P. and Diamond, J.M. (1989). Specific regulation of intestinal nutrient transporters by their dietary substrates. Annu.Rev.Physlol. 51, 125-141.
- Höfer, D., Püschel, B. and Drenckhahn, D. (1996). Taste receptor-like cells in the rat gut identified by expression of α-gustducin. Proc. Nat. Acad. Sci USA 93, 6631- 6634.
- Karasov, W.H. and Diamond, J.M. (1987) in Physiology of the Gastrointestinal tract (Johnson, L.R., ed.), pp. 1489-1497, Raven Press, New York.
- Kitagawa, M., Kusakabe, Y., Miura, H., Niromiya, Y. and Hino, A. (2001). Molecular genetic identification of a candidate receptor gene for sweet taste. Biochem Biophys. Res. Comm. 283, 236-242.
- Lescale-Matys, L., Dyer, J., Scott, D., Freeman, T.C., Wright, E.M. and Shirazi-Beechey, S.P. (1993). Regulation of the ovine intestinal Na+/glucose co-transporter (SGLT1) is dissociated from mRNA abundance. Biochem. J. 91, 435-440.
- Max, M., Shanker, Y.G., Hung, L., Rang, M., Liu, Z., Campagne, F, Weinstein, H., Damak, S. and Margolskee, R.F. (2001). Tas 1r3, encoding a new candidate taste receptor, is allelic to the sweet responsiveness Locus Sac. Nature genetics 28,58-63
- Meddings J.B., DeSouza D., Goel M., Thiesen S. (1990). Glucose transport and microvillus membrane physical properties along the crypt-villus axis of the rabbit. J Clin Invest, 85, 1099-1107.
- Roberts A, Renwick AG, Sims J, Snodin DJ. Sucralose metabolism and pharmakinetics in man. Food Chem Toxicol. 2000; 38 (Suppl 2) S31-S41.
- Ruiz-Avila, L., Wong, G.T., Damak, S. and Margolskee, R.F. (2001). Dominant loss of responsiveness to sweet and bitter compounds caused by a single mutation in α-gustducin. Proc. Natl. Acad. Sci USA 98, 8868-8873.
- Shirazi-Beechey, SP (1996) Proc. Nutr. Soc. 55, 167-178.
- Shirazi-Beechey SP, Davies AG, Tebbutt K, Dyer J, Ellis A, Taylor CJ, et. al. (1990). Preparation and properties of brush-border membrane vesicles from human small intestineGastroenterology, 98, 676-685.
- Solberg, DH and Diamond, JM (1987). Comparison of different dietary sugars as inducers of intestinal sugar transporters. Am. J. Physiol. 252, G574-G584.
- Taniguchi, K. (2004) Expression of the sweet receptor protein, T1R3, in the human liver and pancreas. Journal of Veterinary Medical Science, 66, 1311-1314.
- Wong, G.T., Gannon, K.S. and Margolskee, R.F. (1996). Transduction of bitter and sweet taste by gustducin. Nature, 381, 737-738.

## Claims

1. The use of an inhibitor of the T1 R2 and/or T1 R3 receptor and/or α-gustducin selected from the list consisting of an antibody binding to the ligand binding domain of the T1R2 and/or T1R3 receptor, a soluble peptide or a peptido-mimetic comprising the ligand binding domain of the receptor and the G352P-α-gustducin dominant negative mutant for the manufacture of a medicament to treat obesity and diabetes by modulating the capacity of the gut to absorb dietary sugars.

2. The use of an isolated intestinal epithelial cell expressing T1R2 and/or T1R3 and/or α-gustducin to screen for compounds for the manufacture of a medicament to treat obesity and/or diabetes.

## Patentansprüche

1. Verwendung eines Inhibitors des T1R2- und/oder T1R3-Rezeptors und/oder von α-Gustducin, wobei der Inhibitor aus der Liste bestehend aus einem Antikörper, der an die Ligandenbindungsdomäne des T1R2- und/oder T1R3-Rezeptors bindet, einem löslichen Peptid oder einem Peptidomimetikum, das die Ligandenbindungsdomäne des Rezeptors umfasst, und der G352P-α-Gustducin-dominant-negativen Mutation ausgewählt ist, zur Herstellung eines Arzneimittels zur Behandlung von Adipositas und Diabetes durch Modulierung der Fähigkeit des Darmkanals, Zucker in der Nahrung zu absorbieren.

2. Verwendung einer isolierten Darmepithelzelle, die T1 R2- und/oder T1 R3 und/oder α-Gustducin exprimiert, zum Screenen auf Verbindungen zur Herstellung eines Arzneimittels zur Behandlung von Adipositas und/oder Diabetes.

## Revendications

1. Utilisation d'un inhibiteur du récepteur T1R2 et/ou T1R3 et/ou d'α-gustducine sélectionné dans la liste constituée d'un anticorps se fixant au domaine de liaison au ligand du récepteur T1 R2 et/ou T1R3, d'un peptide soluble ou d'un agent peptido-mimétique comprenant le domaine de liaison au ligand du récepteur et le mutant négatif dominant G352P-α-gustducine pour la fabrication d'un médicament pour traiter l'obésité et le diabète en modulant la capacité de l'intestin à absorber des sucres alimentaires.

2. Utilisation d'une cellule épithéliale intestinale isolée exprimant T1 R2 et/ou T1R3 et/ou l'α-gustducine pour cribler pour des composés pour la fabrication d'un médicament pour traiter l'obésité et/ou le diabète.
